# EUROPEAN PATENT APPLICATION

(11) **EP 2 543 363 A1**
(43) Date of publication of application: **09.01.2013**
(21) Application number: 11305875.4
(22) Date of filing: 07.07.2011
(51) Int. Cl.: A61K 9/16, A61K 9/50, A61K 31/343

(54) **Sustained release pharmaceutical oral solid dosage forms of dronedarone or one of its pharmaceutically acceptable salts**

(71) Applicant: SANOFI, 75008 Paris (FR)
(72) Inventor: The designation of the inventor has not yet been filed
(74) Representative: Bouron, Estelle

(57) **Abstract**

The instant invention relates to a sustained release pharmaceutical oral solid dosage form comprising a therapeutically effective amount of dronedarone or pharmaceutically acceptable salts thereof adapted to release over a predetermined time period according to a profile of dissolution characterized in that the total amount of dronedarone or pharmaceutically acceptable salts thereof is liberated at 240 minutes.

Preparation and application in therapeutics.

## Description

The instant invention relates to sustained release pharmaceutical oral solid dosage forms of dronedarone or one of its pharmaceutically acceptable salts, to their preparation and to their application in therapeutics.

In recent years, the clinical utility of drugs can be improved with sustained-release formulation by means of improved therapeutics effect, reduced incidence of adverse effects and simplified dosing regimens.

2-n-Butyl-3-[4-(3-di-n-butylaminopropoxy)benzoyl]-5-methylsulphonamidobenzofuran, or dronedarone, and pharmaceutically acceptable salts thereof are described in European Patent EP 0 471 609 B1.

Dronedarone blocks potassium, sodium and calcium channels and also has anti-adrenergic properties.

Dronedarone is an anti-arrhythmic that is effective in maintaining sinus rhythm to prevent recurrence of atrial fibrillation or to lower ventricular rate as well as prevention of cardiovascular hospitalizations or death in patients presenting atrial fibrillation or atrial flutter.

Currently, a dosage regimen of dronedarone of 400 mg in base form equivalent twice a day is employed using immediate release tablets. This may be undesirable at least because patient compliance decreases as the frequency of taking a drug increases.

There is therefore an existing need for a once a day modified release formulation comprising dronedarone or one of its pharmaceutically acceptable salts with reliable slower absorption over a targeted period of time.

Thus a subject of the invention is a sustained release pharmaceutical oral solid dosage form comprising a therapeutically effective amount of dronedarone or pharmaceutically acceptable salts thereof adapted to release over a predetermined time period according to a profile of dissolution characterized in that the total amount of dronedarone or pharmaceutically acceptable salts thereof is liberated at 240 minutes.

Another subject of the invention is a sustained release pharmaceutical oral solid dosage form comprising a therapeutically effective amount of dronedarone or pharmaceutically acceptable salts thereof adapted to release over a predetermined time period according to a profile of dissolution characterized in that the total amount of dronedarone or pharmaceutically acceptable salts thereof is liberated at 240 minutes, said dissolution profile is measured after introducing said sustained release pharmaceutical oral solid dosage form in a solution at pH = 4,5 comprising potassium dihydrogen orthophosphate in 1000 ml of water.

Another subject of the invention is a sustained release pharmaceutical oral solid dosage form comprising a therapeutically effective amount of dronedarone or pharmaceutically acceptable salts thereof adapted to release over a predetermined time period according to a profile of dissolution characterized in that 5 to 40% of dronedarone or pharmaceutically acceptable salts thereof is liberated at 30 minutes and the total amount of dronedarone or pharmaceutically acceptable salts thereof is liberated at 240 minutes.

Another subject of the invention is a sustained release pharmaceutical oral solid dosage form comprising a therapeutically effective amount of dronedarone or pharmaceutically acceptable salts thereof adapted to release over a predetermined time period according to a profile of dissolution characterized in that around 30 % of dronedarone or pharmaceutically acceptable salts thereof is liberated at 30 minutes and the total amount of dronedarone or pharmaceutically acceptable salts thereof is liberated at 240 minutes.

Another subject of the invention is a sustained release pharmaceutical oral solid dosage form comprising a therapeutically effective amount of dronedarone or pharmaceutically acceptable salts thereof adapted to release over a predetermined time period according to a profile of dissolution characterized in that the total amount of dronedarone or pharmaceutically acceptable salts thereof is liberated at 240 minutes and in that it comprises a release controlling agent.

Another subject of the invention is a sustained release pharmaceutical oral solid dosage form comprising a therapeutically effective amount of dronedarone or pharmaceutically acceptable salts thereof adapted to release over a predetermined time period according to a profile of dissolution characterized in that 5 to 40 % of dronedarone or pharmaceutically acceptable salts thereof is liberated at 30 minutes and the total amount of dronedarone or pharmaceutically acceptable salts thereof is liberated at 240 minutes and in that it comprises a release controlling agent.

Another subject of the invention is a sustained release pharmaceutical oral solid dosage form comprising a therapeutically effective amount of dronedarone or pharmaceutically acceptable salts thereof adapted to release over a predetermined time period according to a profile of dissolution characterized in that around 30 % of dronedarone or pharmaceutically acceptable salts thereof is liberated at 30 minutes and the total amount of dronedarone or pharmaceutically acceptable salts thereof is liberated at 240 minutes and in that it comprises a release controlling agent.

Said release controlling agent may be selected from the group comprising cellulose derivatives.

Among the pharmaceutically acceptable salts of dronedarone, mention may be made of the hydrochloride.

Said sustained-release pharmaceutical oral solid dosage form may also comprise a pharmaceutically acceptable non ionic hydrophilic surfactant such as polyoxyethylene-polyoxypropylene copolymers also called poloxamers, more particularly poloxamer 407 particularly in a range between 1% to 20% of the amount of dronedarone as base form, particularly in a range between 5% to 15% of the amount of dronedarone as base form and more particularly at 10% the amount of dronedarone as base form and/or in a range between 5 and 6% of the total amount of dosage form and more particularly around 5.5%.

Said release controlling agent may be selected from the group comprising cellulose derivatives such as ethylcellulose and more particularly in a range between 14% and 21% of the total amount of dosage form.

Mention may be made of ethylcellulose 20 mPa.s which may be defined as ethylcellulose with a viscosity of 20 mPa.s.

In one embodiment, the invention may additionally comprise other cellulose derivatives such as hydroxypropylmethylcellulose, more particularly between 3 and 4% of the total amount of dosage form and/or cellulose microcrystalline between 10 and 16% of the total amount of dosage form.

Mention may be made of hydroxypropyl methyl cellulose 6 mPa.s which may be defined as hydroxypropyl methyl cellulose with a viscosity of 6 mPa.s.

Mention may be made of cellulose microcrystalline 90 which may be defined as cellulose with a nominal mean particle size of 90 µm.

In another embodiment, the invention may additionally comprise polymers of N-vinyl-2-pyrrolidone such as crospovidone, more particularly in a range between 6% and 7% of the total amount of dosage form.

Said sustained release pharmaceutical oral solid dosage form may be diffusion-controlled coated formulations.

Said sustained release pharmaceutical oral solid dosage form may be in the form of multiple units administered for example in a sachet.

The sustained release pharmaceutical oral solid dosage form according to the invention may also comprise others excipients for example binder such as maize starch, diluent such as monohydrate lactose, plasticizer such as triethyl citrate, anionic surfactant such as sodium laurylsulfate, emulsifying agent such as cetyl alcohol.

Said excipients are chosen according to the pharmaceutical form and the administration route desired, among usual excipients known of one of skill in the art.

The sustained release pharmaceutical oral solid dosage forms according to the invention can be prepared by use of well known pharmaceutical techniques such as blending, granulation, milling, extrusion-spheronisation or coating.

List of figures:
Figure 1 shows dissolutions profiles of current dronedarone 400 mg immediate release tablet;
Figure 2 shows dissolutions profiles of examples according to the invention.

The following examples describe the synthesis of some dosage forms according to the invention. These examples are not intended to be limitative and only illustrate the present invention.

To examplify the interest of proposed pharmaceutical compositions in the obtention of a form containing a high dose of dronedarone (i.e. 800mg) allowing a once a day administration, various examples are presented hereafter.

### EXAMPLE : Manufacture of multiple units

### 2.10 Microgranules W

In a high shear bottom driven granulator Fielder GP1 type, 1492 g of dronedarone hydrochloride, 91.038 g of hypromellose 6mPa.s, 159.099 g of mais starch, 171.033 g of crospovidone, 140.025 g of poloxamer 407 and 146.147 g of lactose monohydrate are blended for 2 min at an impeller speed of 400 rpm and a chopper speed of 3000 rpm.

Then 550 g of purified water is added quickly on the previous blend under an impeller speed of 200 rpm and a chopper speed of 1500 rpm. Then granulation is run for 1 min using the same impeller and chopper speeds.

Then, using an extruder Fuji-Paudal type, the wet mass is passed through a 1.2 mm screen at a speed of the screw of 40 rpm.

Then extrudates are put in a spheroniser Fuji-Paudal type and using a rotation speed of the plate of 1300 rpm, an air pressure of 2.5 bar and a duration of 1 min 15 s, spheronised granules are obtained.

Then spheronised microgranules are dried in an oven at 40°C overnight. Dried microgranules are sieved on 1.6 mm and 0.800 mm sieves and fraction between 0.800 and 1.6 mm is kept.

Immediate microgranules are called **microgranules W.**

| microgranules W | |
|---|---|
| Components | Amount (mg) |
| Dronedarone hydrochloride | 852 |
| Poloxamer | 80 |
| Hydroxypropyl methyl cellulose 6 mPa.s | 52 |
| Maize starch | 91 |
| Crospovidone type A | 97.5 |
| Monohydrate lactose | 83.3 |
| Uncoated microgranule mass | 1255.8 |

### 2.11 Microgranules X

A coating suspension is prepared as follows: 638 g of ethyl cellulose aqueous suspension and 47.451 g of triethyl citrate are mixed under low rotation speed with 132 g of purified water.

Then 650 g of microgranules **W** is coated with the suspension using a fluidised bed equipment Aeromatic MP1 at a spray rate of 7.5 g/min, inlet air temperature from 52.2 to 62.2°C, outlet air temperature from 41.7 to 46.1°C, air flow rate from 99 to 140 m³/h until a dry deposit of 25.57 per cent of the initial immediate microgranule weight is reached. Coated microgranules are called **X.**

| microgranules X | |
|---|---|
| Components | Amount (mg) |
| Dronedarone hydrochloride | 852 |
| Poloxamer | 80 |
| Hydroxypropyl methyl cellulose 6 mPa.s | 52 |
| Maize starch | 91 |
| Crospovidone type A | 97.5 |
| Monohydrate lactos | 83.3 |
| Uncoated microgranule mass | 1255.8 |
| Triethyl citrate | 62.3 |
| Ethyl cellulose 20 mPa.s | 226.9 |
| Sodium laurylsulfate | 10.9 |
| Cetyl alcohol | 21 |
| Coated microgranule mass | 1577 |

### 2.12 Microgranules Y

In a high shear bottom driven granulator Fielder GP1 type, 1704 g of dronedarone hydrochloride, 336 g of microcrystalline cellulose 90 and 159.991 g of poloxamer 407 are blended for 2 min at an impeller speed of 400 rpm and a chopper speed of 3000 rpm.

Then 550 g of purified water is added quickly on the previous blend under an impeller speed of 200 rpm and a chopper speed of 1500 rpm; Then granulation is run for 1 min using the same impeller and chopper speeds.

Then, using an extruder Fuji-Paudal type, the wet mass is passed through a 1.2 mm screen at a speed of the screw of 40 rpm.

Then extrudates are put in a spheroniser Fuji-Paudal type and using a rotation speed of the plate of 1300 rpm, an air pressure of 2.5 bar and a duration of 1 min 15 s, spheronised granules are obtained.

Then spheronised microgranules are dried in an oven at 40°C overnight. Dried microgranules are sieved on 1.6 mm and 0.800 mm sieves and fraction between 0.800 and 1.6 mm is kept.

Immediate microgranules are called **Y.**

| microgranules Y | |
|---|---|
| Components | Amount (mg) |
| Dronedarone hydrochloride | 852 |
| Poloxamer | 80 |
| Microcrystalline cellulose 90 | 168 |
| Uncoated microgranule mass | 1100 |

### 2.13 Microgranules Z1, Z2, Z3 and Z4

A coating suspension is prepared as follows: 677 g of ethyl cellulose aqueous suspension and 50.303 g of triethyl citrate are mixed under low rotation speed with 140 g of purified water.

Then 650 g of microgranules **Y** is coated with the suspension using a fluidised bed equipment Aeromatic MP1 at a spray rate of 7.9 g/min, inlet air temperature from 53 to 60.8°C, outlet air temperature from 40.2 to 45.9°C, air flow rate from 107 to 140 m³/h until

Samples are collected throughout the coating after a dry deposit of 25.3% (called Z1) - 30% (called Z2) - 36.7% (called Z3) and 39.5% (called Z4) of the initial immediate microgranule weight.

**Table 3 Multiples forms examples (microgranules) - Unit formula**

| Formulations | Z1 | Z2 | Z3 | Z4 |
|---|---|---|---|---|
| Components | mg | mg | mg | mg |
| Dronedarone hydrochloride | 852 | 852 | 852 | 852 |
| Poloxamer | 80 | 80 | 80 | 80 |
| Microcrystalline cellulose 90 | 168 | 168 | 168 | 168 |
| Uncoated microgranule mass | 1100 | 1100 | 1100 | 1100 |
| Triethyl citrate | 54 | 64 | 78.4 | 84.3 |
| Ethyl cellulose 20 mPa.s | 196.7 | 232.7 | 285.2 | 307 |
| Sodium laurylsulfate | 9.5 | 11.2 | 13.7 | 14.7 |
| Cetyl alcohol | 18.3 | 21.6 | 26.4 | 28.4 |
| Coated microgranule mass | 1378.5 | 1429.5 | 1503.7 | 1534.4 |

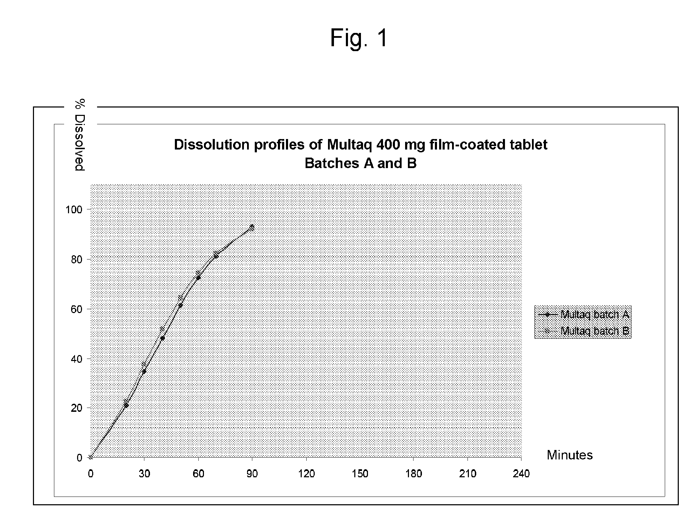

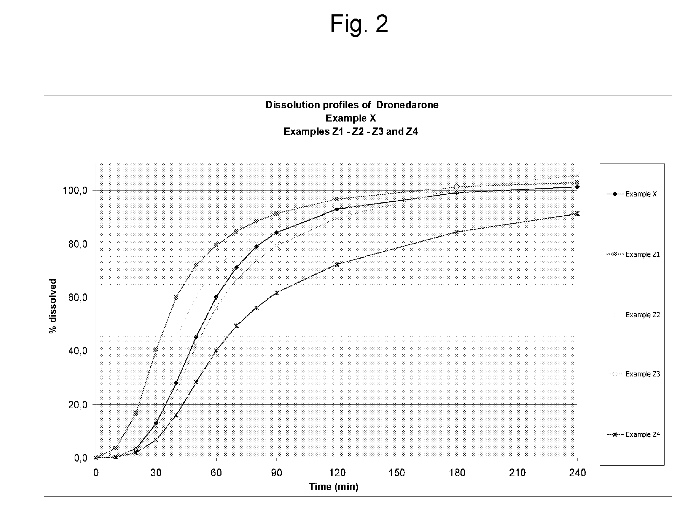

Dissolution profiles of immediate release tablet and examples using dissolution methods described hereafter are presented in figures 1 and 2.

Dissolution method description for current dronedarone 400 mg immediate

### release tablet

This dissolution method proceed according to the dissolution test for solid oral dosage forms (Ph. Eur. 2.9.3 and USP <711>) using a paddle apparatus.

The dissolution medium is prepared by dissolving 13.61 mg of potassium dihydrogen orthophosphate in 1000 ml of ultra-purified water. If necessary, adjust the pH to 4.50 ± 0.05 with 0.1 M hydrochloric acid or 0.1 M sodium hydroxide.

The experimental conditions are:
- Introduce 1000 ± 10 ml of dissolution medium into each vessel
- Set the temperature to 37 ± 0.5°C and the paddle speed at 75 rpm.

The reference solution is prepared by dissolving 42.6 mg of dronedarone hydrochloride working standard in 2.0 ml of methanol and dilute to 100.0 ml with dissolution medium.

The procedure comprises the following steps:
- Introduce one tablet, into each of six dissolution vessels and immediately start the paddles.
- Using an automatic system, sample the medium at 10, 20, 30, 40, 50, 60, 70, 80 and 90 minutes.
- Filter the samples through glass filters 10µm (Whatman GF/D, or equivalent) or polypropylene filters (Prolabo, or equivalent).
- Determine the dronedarone hydrochloride spectrophometrically at 288 nm using a 1 mm quartz cell and dissolution medium as the blank.
- Measure the absorbance of the reference solution under the same conditions.

### Dissolution method description for the examples

This dissolution method proceed according to the dissolution test for solid oral dosage forms (Ph. Eur. 2.9.3 and USP <711 >) using a paddle apparatus.

The dissolution medium is prepared by dissolving 13.61 mg of potassium dihydrogen orthophosphate in 1000 ml of ultra-purified water.

If necessary, adjust the pH to 4.50 ± 0.05 with 0.1M hydrochloric acid or 0.1M sodium hydroxide.

The experimental conditions are:
- Introduce 1000 ± 10 ml of dissolution medium into each vessel.
- Set the temperature to 37 ± 0.5°C and the paddle speed at 75 rpm.

The reference solution is prepared by dissolving 85.2 mg of dronedarone hydrochloride working standard in 2.0 ml of methanol and dilute to 100.0 ml with dissolution medium.

The procedure comprises the following steps:
- Introduce a quantity of multiple form product equivalent to 800mg base of active product, into each of three dissolution vessels and immediately start the paddles.
- Using an automatic system, sample the medium at 10, 20, 30, 40, 50, 60, 70, 80, 90, 120, 180 and 240 minutes.
- Filter the samples through glass filters 10µm (Whatman GF/D, or equivalent) or polypropylene filters (Prolabo, or equivalent).
- Determine the dronedarone hydrochloride spectrophometrically at 264 nm using a 1 mm quartz cell and dissolution medium as the blank.
- Measure the absorbance of the reference solution under the same conditions.

In the sustained release pharmaceutical oral solid dosage form according to the invention, dronedarone or a pharmaceutically salt thereof can be administered as a unitary dosage form, in blend with usual pharmaceutical excipients, to animals and human beings for the prevention or for the treatment of diseases mentioned above.

By the oral route, the dose of active principle to administer per day can reach 800 mg.

In specific cases, higher or lower dosages may be appropriate; these dosages are comprised within the scope of the present invention. According to usual practice, the dosage suitable to each patient is determined by the physician according to the administration route, the weight and response of the patient.

The present invention, according to another of its aspects, also relates to a method for the treatment of the above pathologies, which comprises the administration to a patient of an effective dose of a dronedarone or a salt with a pharmaceutically acceptable salt thereof.

## Claims

1. Sustained release pharmaceutical oral solid dosage form comprising a therapeutically effective amount of dronedarone or pharmaceutically acceptable salts thereof adapted to release over a predetermined time period according to a profile of dissolution **characterized in that** the total amount of dronedarone or pharmaceutically acceptable salts thereof is liberated at 240 minutes.

2. Dosage form according to claim 1 **characterized in that** said dosage form comprises a release controlling agent which is selected from cellulose derivatives.

3. Dosage form according to claim 2 **characterized in that** said cellulose derivatives is ethylcellulose.

4. Dosage form according to anyone of the preceding claims **characterized in that** it also comprises a pharmaceutically acceptable non ionic hydrophilic surfactant.

5. Dosage form according to claim 4 **characterized in that** said pharmaceutically acceptable non ionic hydrophilic surfactant is polyoxyethylene-polyoxypropylene copolymers also called poloxamers.

6. Dosage form according to anyone of the preceding claims **characterized in that** pharmaceutically acceptable salts of dronedarone is the hydrochloride.

7. Dosage form according to anyone of the preceding claims **characterized in that** it additionally comprises hydroxypropylmethylcellulose, and/or cellulose microcrystalline.

8. Dosage form according to anyone of the preceding claims **characterized in that** it additionally comprises polymers of N-vinyl-2-pyrrolidone such as crospovidone.

9. Dosage form according to anyone of the preceding claims **characterized in that** said polymers of N-vinyl-2-pyrrolidone are crospovidone.

10. Dosage form according to anyone of the preceding claims **characterized in that** said dosage form is a diffusion-controlled coated formulations.

11. Dosage form according to anyone of the preceding claims **characterized in that** said sustained release pharmaceutical oral solid dosage form is in the form of multiple units.

12. Medicament, comprising a dosage form according to claims 1 to 11.

13. Dosage form according to claims 1 to 11 for use in the treatment of atrial fibrillation or the prevention of recurrence of atrial fibrillation or death or cardiovascular hospitalization.
